# EUROPEAN PATENT APPLICATION

(11) **EP 4 160 551 A1**
(43) Date of publication of application: **05.04.2023**
(21) Application number: 21306348.0
(22) Date of filing: 29.09.2021
(51) Int. Cl.: G06V 20/56, G06V 20/59, G06V 10/82, G06V 40/18, G06V 20/20, G06V 40/10, G06V 20/40

(54) **METHODS AND SYSTEMS FOR VEHICLE-ASSISTED FEATURE CAPTURE**

(71) Applicant: Société BIC, 92110 Clichy (FR)
(72) Inventor: Duffy, David, 8003 Zurich (CH); Elliott-Bowman, Bernadette, Leatherhead, Surrey, KT22 8LR (GB)
(74) Representative: Peterreins Schley

(57) **Abstract**

The present invention relates to a computer-implemented method for capturing a feature of interest observed by a passenger of a vehicle, comprising detecting an excited level of the passenger's interest based on passenger data generated by monitoring the passenger of the vehicle, thereby generating an interest event; extracting a gaze direction of the passenger relating to the interest event based on the passenger data; and extracting environmental data, generated by monitoring an environment of the vehicle, that relate to the interest event and to the gaze direction, thereby generating partial feature data for the feature of interest observed by the passenger. The present invention further relates to a computer system configured to execute the computer-implemented method for capturing a feature of interest observed by a passenger of a vehicle.

## Description

### TECHNICAL FIELD

This specification relates to a computer-implemented methods, a computer system, and/or a vehicle for capturing a feature of interest observed by a passenger of a vehicle.

### BACKGROUND

Self-driving vehicles of all levels of autonomy utilize various advanced sensors. These include LIDAR and other scanning technologies which analyze the space around the car using signals reflected from physical objects in the environment. These reflected signals may be represented in point clouds and can be automatically processed by the vehicle to locate and identify features. Self-driving vehicles typically generate (360°) point cloud scans that gather sufficient information to enable safe navigation, but usually do not capture all features in detail. LIDAR is moving from passive scanning (simple object detection) to active scanning (intelligent classification and adaptive scan characteristics). Autonomous vehicles may also incorporate sensors inside the cabin to monitor passengers tracking their attention, gaze and emotions.

Point cloud data may be algorithmically reconstructed. Generative adversarial networks (GANs) can be used to predict and fill in incomplete point cloud scans, thereby enabling a complete 3D model of the object. Some systems are known to reproduce even fine features of an object that were missed by the original sparsely populated point cloud scan.

### SUMMARY

According to a first aspect, there is provided a computer-implemented method for capturing a feature of interest observed by a passenger of a vehicle. The method comprises detecting an excited level of the passenger's interest based on passenger data generated by monitoring the passenger of the vehicle, thereby generating an interest event. The method further comprises extracting a gaze direction of the passenger relating to the interest event based on the passenger data. The method further comprises extracting environmental data, generated by monitoring an environment of the vehicle, that relate to the interest event and to the gaze direction, thereby generating partial feature data for the feature of interest observed by the passenger.

According to a second aspect, there is provided a computer system configured to execute the computer-implemented method of the first aspect (or an embodiment thereof) for capturing a feature of interest observed by a passenger of a vehicle.

According to a third aspect, there is provided a vehicle comprising an environment monitoring system configured to monitor an environment of the vehicle, thereby generating environmental data. The vehicle further comprises a passenger monitoring system configured to monitor the passenger of the vehicle, thereby generating passenger data. The passenger monitoring system comprises an emotion detection system configured to detect an excited level of the passenger's interest based on the passenger data, thereby generating an interest event. The passenger monitoring system further comprises a gaze tracking system configured to extract a gaze direction of the passenger relating to the interest event based on the passenger data. The vehicle is configured to couple to or comprises the computer system of the second aspect (or an embodiment thereof).

According to a fourth aspect, there is provided a computer program configured to execute the computer-implemented method of the first aspect (or an embodiment thereof) for capturing a feature of interest observed by a passenger of a vehicle.

According to a fifth aspect, there is provided a computer-readable medium or signal storing the computer program of the fourth aspect (or an embodiment thereof).

Dependent embodiments of the aforementioned aspects are given in the dependent claims and explained in the following description, to which the reader should now refer.

Methods and/or systems of the aforementioned aspects of this specification are directed to capturing a feature of interest observed by a passenger (or user) of a vehicle. In fact, the passenger may glimpse objects or scenes that interest her/him while travelling in the vehicle but may not be able to view them in enough detail to enjoy at the time or to enable them to recreate an accurate sketch later. Self-driving vehicle scans will only collect sufficient data from objects in their environment to enable safe navigation, not to recreate details of those objects.

As disclosed hereinafter, interesting environmental features briefly observed during a vehicle journey may accurately be recreated in 3D using partial point cloud data gathered by e.g. a self-driving vehicle. One or more features may be selected for reproduction by e.g. a GAN based on vehicle-detected user attention and emotion relative to the travel path, and recreated at the user's convenience for modification or enjoyment using an appropriate technology (e.g smartphone, projections, augmented reality etc.). The vehicle scan profile may also be adapted in real time to gather further information on a feature when passenger attention is detected. This may include focusing a LIDAR scan on regions of the object of interest that are more complex for the user or GAN to reproduce.

A scenario may be as follows: A passenger rides in an autonomous vehicle and observes something outside that they find creatively interesting or inspiring. The vehicle detects the passenger's interest via its passenger monitoring sensors and extracts their gaze at the time of the observation. The system uses the gaze information to determine where the passenger was looking at the time of the observation. The passenger's gaze direction is used to define and extract matching point cloud (or other) data from the vehicle's external sensors that can be used to reconstruct a digital representation of the feature of interest later. The system may collect further object data using adaptive scan characteristics of the vehicle sensor system, using the user's gaze to direct the scan path. Later, the passenger may view the digital reconstruction of the feature they found interesting via, for example, an augmented reality smartphone interface. The passenger may use the reconstruction to inspire her/his creativity.

A/the computer system may be configured to detect and recreate physical features that a user has observed during a vehicle journey and experienced a positive emotional or other response as a result. A passenger's emotional response and gaze direction when viewing a feature of interest external to the vehicle may be used to determine the position of the feature relative to the vehicle's external sensors and extract sensor data (primarily, point cloud data) relevant to that feature. Passenger's emotion and gaze data may be used to actively direct the scan path of a vehicle's external sensors towards features of interest in the local environment. The system may learn the user's environmental feature preferences over time and pre-emptively apply a directed scan path from a vehicle to collect the maximum amount of sensor data from preferred upcoming objects during a journey.

According to this specification a user/passenger is enabled to view reconstructed models of personal features of interest that she/he has observed during a journey in a vehicle. The vehicle's sensors may be used to determine when an interesting feature has been observed and to collect partial sensor data on that feature that can later be used to inform a digital reconstruction of the feature. Providing digital reconstructions allows passengers to fully appreciate interesting features that they have briefly glimpsed while travelling in a vehicle 300 but could not view in detail at the time due to the constraints of the journey. Features of interest may be detected automatically, allowing the system to record the relevant feature information. Within the travel timescales of the journey, rapid additional data collection using vehicle sensors at the time the feature was observed may be enabled. Without requiring any additional effort from the user, the passenger is allowed to continue enjoying their journey and observing further features. Passengers may use reconstructed features to inspire a creative mindset, for educational purposes or to act as an accurate 3D model for sketching or painting the feature of interest.

### FIGURE DESCRIPTION

**Fig. 1** schematically illustrates a computer-implemented method for capturing a feature of interest observed by a passenger of a vehicle.
**Fig. 2** schematically illustrates an exemplary embodiment of the computer-implemented method for capturing a feature of interest observed by a passenger of a vehicle.
**Fig. 3** illustrates an exemplary architecture of a computer system configured to run the computer-implemented method for capturing a feature of interest observed by a passenger of a vehicle.
**Fig. 4** illustrates an exemplary flow chart for a computer system configured to run the computer-implemented method for capturing a feature of interest observed by a passenger of a vehicle.
**Fig. 5** illustrates a vehicle 300 configured to execute the computer-implemented method for capturing a feature of interest observed by a passenger of a vehicle.

### DETAILED DESCRIPTION

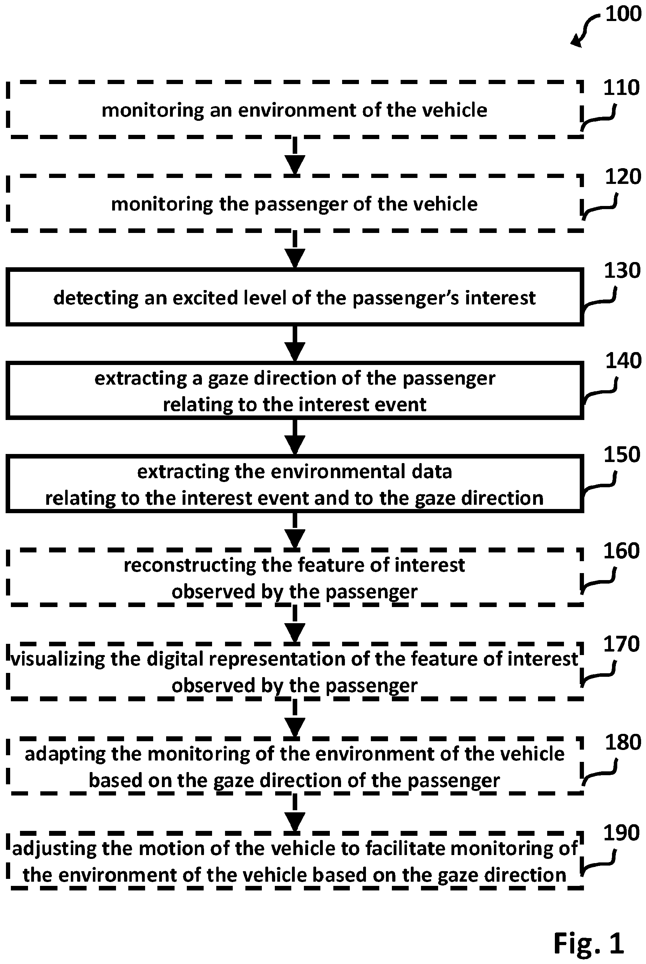

The computer-implemented method 100 for capturing a feature of interest observed by a passenger of a vehicle 300 may or may not comprise monitoring 110 an environment of the vehicle 300, thereby generating environmental data. The method 100 may or may not comprise monitoring 120 the passenger of the vehicle 300, thereby generating passenger data. The method 100 further comprises detecting 130 an excited level of the passenger's interest (at least) based on (the) passenger data generated by monitoring 120 the passenger of the vehicle 300, thereby generating an interest event. The method 100 further comprises extracting 140 a gaze direction of the passenger relating to the interest event (at least) based on the passenger data. The method 100 further comprises extracting 150 (the) environmental data, generated by monitoring (110) an environment of the vehicle 300, that relate to the interest event and to the gaze direction, thereby generating partial feature data for the feature of interest observed by the passenger. The computer-implemented method 100 is schematically illustrated in **Fig. 1****.** For example, the method 100 may comprise steps 130, 140, and 150. The method 100 may or may not comprise step 110. The method 100 may or may not comprise step 120. The method 100 may or may not comprise step 160. The method 100 may or may not comprise step 170. The method 100 may or may not comprise step 180. The method 100 may or may not comprise step 190. Steps 110 and/or 120 may be carried out independently of the method 100, e.g. by an electronic control unit (ECU) of an autonomous driving control system of the vehicle 300. As an example, the order of steps 110 and 120 is immaterial. Steps 180 may be carried out at any time after step 140 and e.g. before step 150, 160, 170, or 190. Steps 190 may be carried out at any time after step 140 and e.g. before step 150, 160, 170, or 180.

The environment of the vehicle 300 may comprise a (planar) angle of 360°, i.e. encircle the vehicle 300 entirely. Furthermore, the environment of the vehicle 300 may be covered by a plurality of solid angles. As an example, the environment of the vehicle 300 may be monitored 110 by means for laser imaging, detection and ranging (LIDAR). Alternatively, or in addition, the environment of the vehicle 300 may be monitored 110 by means of radio detection and ranging (RADAR). Alternatively, or in addition, the environment of the vehicle 300 may be monitored 110 by means of a camera system configured to capture the environment of the vehicle 300 in the ultraviolet, in the visible spectrum, and/or in the infrared. Any scanning technology which analyzes the environment of the vehicle 300 (based on external sensors of the vehicle) and produces a point cloud output, or any combinations of such scanning technologies may be used. Monitoring 110 the environment of the vehicle 300 may be implemented and carried out by an environment monitoring system of the vehicle 300. Monitoring 110 the environment of the vehicle 300 may be (quasi-)continuous while the vehicle 300 is in use and, in particular, while the vehicle 300 is driving.

The vehicle 300 within which the passenger may travel from one location to another may be a partly autonomous vehicle 300 (levels 1 to 4) or an (fully) autonomous vehicle 300 (level 5). Such a vehicle 300 is typically equipped with scanning technologies for monitoring 110 the environment of the vehicle 300 to enable safe navigation. The vehicle 300 must not necessarily be autonomous (levels 1 to 5) provided that the vehicle 300 still comprises a suitable environment monitoring system. On the other hand, the vehicle 300 being autonomous (levels 1 to 5) is beneficial as in this case monitoring 110 the environment of the vehicle 300 needs to be carried out anyway. In fact, in a typical autonomous scenario relevant for this disclosure at least one passenger may sit in the vehicle 300 as it autonomously navigates through a space according to instructions given to a navigation system of the vehicle 300. As an example, the vehicle 300 may use the environment monitoring system generating the environmental data to observe its environment and detect physical features around which to navigate. The driver of the vehicle 300 of a non-autonomous vehicle 300 gradually turns into a mere passenger, the higher the level of automation of the vehicle 300.

The environmental data may comprise point cloud data. Here a point cloud may be a set of points in a space that may be higher-dimensional (e.g. a space of three or more dimensions). Each point may relate to a point of time. A point in a 3D point cloud may represent a location in the environment of the vehicle 300. As an example, the environmental data may comprise (or be) a time series of data. Such a time series of data may result from (quasi-)continuous monitoring 110. For each point in time of the time series the data may comprise (or be) a point cloud, that is a set of points in a space representing the environment of the vehicle 300. The environmental data may typically comprise partial point cloud data given that usually only few perspectives of the environment are captured.

The feature of interest observed by the passenger may be any physical phenomenon (e.g. a scene, lighting ...) in the environment of the vehicle 300 that may be imaged by the passenger and/or the vehicle 300. Alternatively, or in addition, the feature of interest observed by the passenger may be directed to a physical object in the environment of the vehicle 300. For example, the physical object may comprise (or be) a person, an animal and/or a plant (a tree, a flower, or the like). Alternatively, or in addition, the physical object may comprise (or be) another means of transportation (another vehicle 300, bicycle, sports car, classic car, or the like). Alternatively, or in addition, the physical object may comprise (or be) an architectural structure (building, a tourist attraction, a monument, a fountain, a square, or the like). Alternatively, or in addition, the physical object may comprise (or be) an incidental object. The aforementioned examples shall not be construed as being limiting. In general, the physical object may be whatever arouses a positive emotional and/or psychological state in the passenger. Examples for such a positive emotional and/or psychological state may comprise one or more of interest, happiness, awe/inspiration, and surprise. In particular, the physical object may be creatively interesting or inspiring for the passenger, that is interest the user for creative purposes. In this case, the passenger may use the reconstructed 160 digital representation of the feature of interest to inspire her/his creativity.

Monitoring 120 the passenger of the vehicle 300 may comprise monitoring the passenger's emotional and/or psychological state, thereby generating passenger state data. Detecting 130 the excited level of the passenger's interest may then (at least) be based on the passenger state data. As an example, monitoring the passenger's emotional and/or psychological state may comprise acquiring passenger state data that may be analyzed based on facial recognition technologies. Such passenger state data may e.g. be acquired from a camera directed towards the face of the passenger. Alternatively, or in addition, monitoring the passenger's emotional and/or psychological state may comprise acquiring passenger state data from one or more galvanic skin response sensors. Alternatively, or in addition, monitoring the passenger's emotional and/or psychological state may comprise monitoring the passenger's heart rate. Alternatively, or in addition, monitoring the passenger's emotional and/or psychological state may comprise monitoring the passenger's respiratory system. Alternatively, or in addition, monitoring the passenger's emotional and/or psychological state may comprise monitoring the passenger's pupillary response. Alternatively, or in addition, monitoring the passenger's emotional and/or psychological state may comprise analyzing the passenger's voice and/or speech. Other user monitoring sensors may be used. Detecting 130 the excited level of the passenger's interest based on the passenger data may comprise analyzing the passenger's heart rate. Alternatively, or in addition, detecting 130 the excited level of the passenger's interest based on the passenger data may comprise analyzing the passenger's respiratory system. Alternatively, or in addition, detecting 130 the excited level of the passenger's interest based on the passenger data may comprise analyzing the passenger's pupillary response. Alternatively, or in addition, detecting 130 the excited level of the passenger's interest based on the passenger data may comprise analyzing the passenger's voice. Alternatively, or in addition, detecting 130 the excited level of the passenger's interest based on the passenger data may comprise analyzing the passenger's speech. In fact, for example, one or more (temporal) changes in the passenger's heart rate, in the passenger's respiratory system, in the passenger's pupillary response, in the passenger's voice, and/or in the passenger's speech may serve as indicator(s) for an "excited" level of the passenger's interest.

As an example, monitoring 120 the passenger of the vehicle 300 may be carried out by a passenger monitoring system. The passenger monitoring system may comprise an emotion detection system which may be configured to monitor (and interpret) the passenger's emotional and/or psychological state.

Monitoring 120 the passenger of the vehicle 300 may be (quasi-)continuous while the vehicle 300 is in use and, in particular, while the vehicle 300 is driving. The passenger data may comprise the passenger state data. The passenger state data may comprise (or be) a time series of data. Such a time series of data may result from (quasi-)continuous monitoring 120. As an example, for each point in time of the time series the data may comprise (or be) a vector, that is a point in a space representing the passenger's emotional and/or psychological state.

Monitoring 120 the passenger of the vehicle 300 may comprise monitoring the passenger's gaze direction, thereby generating passenger gaze data. Extracting 140 the gaze direction of the passenger relating to the interest event may then (at least) be based on the passenger gaze data. Apart from measuring the gaze direction monitoring the passenger's gaze direction may also be used for determining an attention level, an alertness, and/or other information of the passenger. As an example, monitoring the passenger's gaze direction may comprise camera tracking of the passenger's pupillary response. Alternatively, or in addition, monitoring the passenger's gaze direction may comprise tracking the passenger's earbud/hearable-detected saccade motions. Other technologies for gaze monitoring may be applied.

The passenger monitoring system may comprise a gaze tracking system which may be configured to monitor the passenger's gaze direction.

The passenger data may comprise the passenger gaze data. The passenger gaze data may comprise (or be) a time series of data. Such a time series of data may result from (quasi-)continuous monitoring 120. As an example, for each point in time of the time series the data may comprise (or be) a vector, that is a point in a space representing the passenger's gaze direction. The vector may indicate the gaze direction of the passenger.

Detecting 130 the excited level of the passenger's interest based on the passenger data may comprise applying the passenger data or a portion thereof relating to a current point in time to a predetermined criterion and detecting the excited level of the passenger's interest relating to the current point in time if the predetermined criterion is satisfied. The interest event may comprise (or relate to) the current point of time. Detecting 130 the excited level of the passenger's interest based on the passenger data may be (quasi-)continuous while the vehicle 300 is in use and, in particular, while the vehicle 300 is driving. For example, the portion of the passenger data relating to the current point may comprise (or be) the passenger data generated for (or at) the current point in time, that is current passenger data. It may suffice to detect 130 the excited level of the passenger's interest (only) based on the current passenger data. On the other hand, passenger data generated at previous times, that is previous passenger data, may be taken into account, thereby allowing comparisons between current and previous passenger data. Here the passenger data (or the portion thereof relating to the current point in time) may comprise the passenger state data (or portions thereof). Alternatively, or in addition, passenger gaze data may be taken into account.

For example, the passenger data may comprise data relating the passenger's heart rate. Alternatively, or in addition, the passenger data may comprise data relating the passenger's respiratory system. Alternatively, or in addition, the passenger data may comprise data relating the passenger's pupillary response. Alternatively, or in addition, the passenger data may comprise data relating the passenger's voice. Alternatively, or in addition, the passenger data may comprise data relating the passenger's speech. The excited level of the passenger's interest may be detected 130, if one or more of the data relating to one or more of the passenger's heart rate, the passenger's respiratory system, the passenger's pupillary response, the passenger's voice, and/or the passenger's speech satisfy the predetermined criterion.

As an example, applying the passenger data (or a portion thereof) to the predetermined criterion may comprise computing a scalar quantity based on the passenger data (or a portion thereof) and checking, whether a pre-determined threshold value is exceeded, thereby defining the notion of an excited level of passenger's interest. As another example, the predetermined criterion may be any classification algorithm configured to classify an excited level of passenger's interest and, in particular, a machine learning algorithm that has been pre-trained based on training data. Such training data may comprise previous passenger data (and corresponding labels). The predetermined criterion may be implemented in terms of an interest monitoring algorithm.

The interest event may comprise (or be) a timestamp corresponding to the current time of time. Alternatively, or in addition, the interest event may comprise (or be) a time interval defined by a first timestamp and a second timestamp that is larger than the first timestamp. Alternatively, or in addition, the interest event may comprise (or be) a vector of increasing timestamps.

As an example, the vehicle 300 may monitor the passenger's comfort, attention and behavior. The (at least one) passenger may observe the environment from inside the vehicle 300. The interest monitoring algorithm may receive a continuous stream of passenger (state) data from the emotion detection system to be continuously analyzed. Upon analyzing the passenger (state) data the monitoring algorithm may detect when, at some point during the journey, the passenger observes something that she/he perceives positively, that is a feature of interest. This detection may be based on recognizing a positive emotional change in the passenger. When a positive change in the passenger (state) data is detected by the interest monitoring algorithm, an interest event with a current timestamp may be issued.

For example, or if need be, one or more filters may be applied to the passenger (state) data such that the number of interest events during a vehicle 300 journey can be reduced. As an example, a filter may filter by the type of emotion/response detected. In so doing only features that produce e.g. surprise in the passenger may be classified as interest events. Alternatively, or in addition, a filter (or another filter) may filter by the intensity of the emotion/response detected. In so doing e.g. only emotions/responses identified as strong/intense may be classified as interest events. Alternatively, or in addition, a filter (or another filter) may filter out false interest events produced by other stimuli, such as speech-induced emotion. This may, for example, be achieved by analyzing the passenger's speech to identify emotional content unrelated to objects outside the vehicle 300 and/or detecting gaze or head motion of the passenger to identify when the passenger's attention is focused inside or outside of the vehicle 300.

Extracting 140 the gaze direction of the passenger relating to the interest event based on the passenger data may comprise selecting a gaze direction from the generated passenger data relating to the current point in time. Here the passenger data may comprise the passenger gaze data. Alternatively, or in addition, passenger state data may be taken into account. The generated passenger data relating to the current point in time may be the generated passenger data at or closest to the current point in time. If the interest event comprises more than one timestamp, a gaze direction from the generated passenger data may be selected at each timestamp of the interest event or as close as possible to each timestamp of the interest event. Such information may help in extracting 150 the environmental data, in particular, when the vehicle 300 is moving.

For example, a gaze vector extraction algorithm may be configured to analyze the passenger gaze data and to determine in which direction the passenger was looking when the interest event occurred. In fact, the interest event may be shared with the gaze vector extraction algorithm, which may use the at least one timestamp of the interest event to request associated passenger gaze data from the gaze tracking system. For example, passenger gaze data may be requested for the specific point of time (the current point of time) at which the interest event occurred, or for a predefined time box covering short periods (e.g. on the order of seconds or milliseconds) before and after the interest event occurred. A time box may be used to indicate or validate an interest event more accurately, such that the passenger may be likely to attempt to keep her/his gaze trained on a feature of interest as the vehicle 300 passes it. This additional information may be used to more accurately indicate the feature of interest relative to the vehicle's position and speed. Furthermore, the definition of the time box may be made relative to the current velocity (direction and speed) of the vehicle 300, which will affect the amount of time over which the passenger would have been able to observe the feature of interest that produced the interest event.

The gaze vector extraction algorithm may analyze the passenger gaze data to determine the passenger's gaze vector. Gaze tracking technologies that are known in the art may be used to determine the gaze vector from the passenger gaze data, where the timestamp of the interest event may be used to indicate the point at which the passenger gaze data should be assessed. The gaze vector may be expressed as, for example, a set of Cartesian coordinates with 0-0-0 axis location centered between the passenger's eyes. As such, the direction of the passenger's gaze may be expressed in a way that can be algorithmically correlated with the environmental data gathered by the environment monitoring system, to identify a corresponding coordinate point or gaze vector outside the vehicle 300. Sensors inside the vehicle 300 may also be used to account for variations in passenger seating position, height and other factors that may adjust the 0-0-0 axis point of a passenger's gaze.

Extracting 150 the environmental data that relate to the interest event and to the gaze direction may comprise selecting a portion from the environmental data relating to the current point in time and (relating to/generated for) the gaze direction. The environmental data relating to the current point in time may be the environmental data at or closest to the current point in time. If the interest event comprises more than one timestamp, portions from the environmental data may be selected at each timestamp of the interest event or as close as possible to each timestamp of the interest event. Furthermore, if a gaze direction from the generated passenger data has been selected at each timestamp of the interest event or as close as possible to each timestamp of the interest event, portions from the environmental data may be selected that have been generated for respective gaze directions. The term "partial" in partial feature data generated in step 150 may allude to the circumstance that the feature of interest is usually not fully captured by the vehicle 300 passing by.

For example, a feature extraction algorithm may be configured to collate and assess environmental data associated with the (at least one) gaze vector. The feature extraction algorithm may use the gaze vector and event timing (e.g. the at least one timestamp of the interest event) to gather associated point cloud scanning data from the environment monitoring system, thereby generating the partial feature data. The partial feature data is likely to be incomplete, as the purpose of the environment monitoring system in its normal state of operation is to enable the vehicle 300 to navigate safely, rather than to collect high-resolution imaging data about all features in its environment.

The feature extraction algorithm may receive the (at least one) gaze vector and generate a request for associated environmental data from the environment monitoring system. The gaze vector and at least one timestamp of the interest event together may indicate to the environment monitoring system when and from which direction the environmental data should be extracted. Such extracted environmental data may be returned to the feature extraction algorithm e.g. as a point cloud comprised of a multitude of data points spatially distributed in a space of at least three dimensions (e.g. three spatial dimensions and one temporal dimension). Alternatively, or in addition, such extracted environmental data may be returned as a time series of point clouds in a space of at least three dimensions. The feature extraction algorithm may assess the quality of the returned environmental data by, for example, an analysis of the point volume in the point cloud, where a higher number of points equates to a higher resolution and thus to a better quality scan. Where the feature of interest is within the passenger's field of vision but outside the range of the environment monitoring system's sensors, the feature extraction algorithm may fail to extract suitable environmental data. Current LIDAR systems are capable of detecting objects from up to 400m away. In such a scenario, the feature reconstruction process may be halted. Alternatively, or in addition, the system may identify the next nearest physical feature to the gaze vector for reconstruction, where the passenger's interest in the selected feature may be verified at a later stage.

The method 100 may further comprise saving the partial feature data of the feature of interest observed by the passenger.

The method 100 may comprise reconstructing 160 the feature of interest observed by the passenger (at least) based on the partial feature data, thereby generating a digital representation of the feature of interest observed by the passenger. Thanks to reconstructing 160 the digital representation of the feature of interest may be more complete than the partial feature data.

Reconstructing 160 the feature of interest observed by the passenger may comprise applying the partial feature data to a pre-trained generative adversarial network (GAN) configured to output the digital representation of the feature of interest observed by the passenger. In general, the digital representation of the feature of interest observed by the passenger may be reconstructed based on a machine learning algorithm that has been pre-trained (using e.g. training data in supervised learning) for the task of reconstructing 160 the digital representation of the feature of interest observed by the passenger.

The method 100 may further comprise saving the digital representation of the feature of interest observed by the passenger.

For example, a system (e.g. referred to as feature display system or creative feature display system) may be configured to construct (and display) the digital representation of the feature of interest observed by the passenger. The (creative) feature display system may comprise an implementation of an algorithm (e.g. referred to as the feature reconstruction algorithm) configured to digitally reconstruct the (incomplete) partial/combined feature data into a/the digital representation of the feature or region of interest observed by the passenger (e.g. referred to as the reconstructed feature). The algorithm may predict or fill in gaps in the partial/combined feature data to enable a fully realized representation of the reconstructed feature that the user can view at a later point. The (creative) feature display system may comprise a user-interface (e.g. referred to as the feature display user interface) configured to display the reconstructed feature to the passenger (or to any user). The user-interface may further be configured to allow the passenger to interact with the reconstructed feature. As an example, such an interaction may comprise choosing a user-defined perspective on the reconstructed feature.

As an example, at some future point of time, the feature reconstruction algorithm may use the partial/combined feature data to generate the reconstructed feature. Both the partial feature data and the combined feature data are likely to remain incomplete, given that the vehicle 300 is moving past the feature of interest and must prioritize driving safely. The reconstructed feature may be produced by applying, for example, a generative adversarial network-enabled (GAN-enabled) point cloud reconstruction technique to the incomplete partial/combined feature data. If need be, additional vehicle 300 sensor data may be used to increase the accuracy of the reconstructed feature. For example, optical/camera-based imaging may by gathered to indicate feature color or shading, as well as provide further detail on small decorative physical features (such as tile patterns, carvings, inscriptions etc.). During the feature reconstruction phase, the feature reconstruction algorithm may also verify the passenger's likely interest in the feature to be reconstructed. This may be achieved by, for example, providing the passenger with a short description or low-resolution preview of the feature that they may use to confirm interest with the system manually and/or using machine vision technologies known in the art to categorize the feature and subsequently compare the categorized feature to a predefined or learned list of features that are known or likely to be of interest to a user for creative purposes. For example, advertisements, billboards, shops and other similar features may cause the passenger to experience states such as surprise or attention that might be classified as an interest event by the interest monitoring algorithm. While the passenger may find these things interesting, they would not typically be defined as features that would be used for e.g. creative purposes by the passenger later. As such, these features may be arbitrarily excluded from reconstruction. Where the feature reconstruction algorithm determines that a non-creative feature has been imaged, the feature reconstruction phase may be abandoned.

The method 100 may comprise visualizing 170 the digital representation of the feature of interest observed by the passenger via a user-interface (e.g. referred to as feature display user interface). As an example, the user-interface may be a smart device (e.g. a smartphone, a tablet, ...) connected to a network (e.g. WiFi, Ethernet, ...) of the vehicle 300. As another example, the user-interface may be a computer comprising a (touch) screen integrated into the vehicle 300. The user-interface may be interactive, that is it may have input/output functionality for the passenger/user. In particular, the digital representation of the feature of interest observed by the passenger may be visualized 170 to the passenger. Alternatively, or in addition, it may be visualized 170 to at least one other passenger of the vehicle 300. The user-interface may or may not be part of the computer system 200.

The digital representation of the feature of interest observed by the passenger may be visualized 170 as a two-dimensional projection. Alternatively, or in addition, the digital representation of the feature of interest observed by the passenger may be visualized 170 as a three-dimensional object. Alternatively, or in addition, the digital representation of the feature of interest may be visualized as an interactive three-dimensional augmented reality object (e.g. for angles of view covered by the monitoring 110 along the motion of the vehicle 300 and/or reconstructed angles of view not covered by the monitoring 110).

For example, the reconstructed feature may be presented to the passenger or any other user via the feature display user interface, where they may use it e.g. to inspire creative work, act as a model for a sketch or painting, or other use cases. Reconstructed feature(s) may be stored in a cloud location or similar, such that the passenger may access them at her/his convenience from an appropriate device. The feature display user interface may display the reconstructed feature to the passenger/user as, for example, an augmented reality object displayed through a smartphone, smart glasses or the like. Alternatively, or in addition, the reconstructed feature may be display to the passenger/user as a light-based image or hologram displayed from a projection device. Alternatively, or in addition, the reconstructed feature may be displayed to the passenger/user as a virtual reality object observed via a VR headset or screen-based virtual environment. Alternatively, or in addition, the reconstructed feature may be display to the passenger/user on other imaging platforms. The feature display user interface may also be used to communicate steps to verify the passenger's interest in a feature.

The method 100 may comprise adapting 180 the monitoring 110 of the environment of the vehicle 300 based on the gaze direction of the passenger, thereby generating additional partial feature data for the feature of interest observed by the passenger. The method 100 may further comprise combining the partial feature data and the additional partial feature data, thereby updating the partial feature data for the feature of interest observed by the passenger.

In other words, by adapting 180 the monitoring 110 of the environment of the vehicle 300 the method 100 may actively direct the scan path of the vehicle's external sensors towards the feature of interest in the environment of the vehicle 300. Such adapting 180 may be beneficial given that e.g. self-driving vehicle 300 scans will typically only collect sufficient data from objects in the environment to enable safe navigation and not to recreate details of those objects.

As an example, the method 100 may comprise computing, based on the gaze direction of the passenger, an angle and/or a solid angle in the environment of the vehicle 300 where the feature of interest is located. The method 100 may further comprise computing a time series of angles and/or solid angles in the environment of the vehicle 300 where the feature of interest is located as the vehicle 300 is moving. As an example, computing the time series of the (solid) angles may be based on the gaze direction of the passenger and the velocity (speed and direction) of the vehicle 300. Alternatively, or in addition, computing the time series of the solid angles may be based on determining a time series of gaze directions of the passenger (including the gaze direction of the passenger extracted in step 140) relating to the interest event based on the passenger data.

As an example, the environment monitoring system may comprise an active scanning capability, whereby the scan path of the sensors of the environment monitoring system may be adaptively adjusted to gather information about specific objects or locations around the vehicle 300. A scan path adjustment algorithm may acquire further scanning data (e.g. referred to as additional partial feature data) from the gaze vector location using the environment monitoring system. For example, the feature extraction algorithm may generate a request (e.g. referred to as additional partial feature data request) for additional partial feature data from the environment monitoring system (e.g. routed through the scan path adjustment algorithm) to improve the quality of the partial feature data. The additional feature data request may be used to actively adjust the scan path of the environment monitoring system to collect targeted additional partial feature data. The scan path adjustment algorithm may request the additional partial feature data from the environment monitoring system, where the additional partial feature data request may be used to define instructions (e.g. referred to as data acquisition instructions) for the environment monitoring system that describe how the additional partial feature data should be acquired. The feature extraction algorithm may algorithmically combine the partial feature data and the additional partial feature data into a single data source (e.g. referred to as combined partial feature data or again as partial feature data) which may be used to reconstruct 160 the feature of interest.

For example, the feature extraction algorithm may generate an additional partial feature data request to collect additional partial feature data from the environment monitoring system. The additional partial feature data request may be handled by the scan path adjustment algorithm and may describe e.g. the gaze vector coordinates, the at least one timestamp of the interest event, and/or the required additional partial feature data quality (e.g. as a function of points per cubic volume). The additional partial feature data request may be triggered e.g. for all interest events, regardless of the partial feature data quality. Alternatively, or in addition, the additional partial feature data request may be triggered e.g. for interest events whereby the partial feature data is of an insufficient quality as assessed against a predefined quality metric (e.g. points per cubic volume). The scan path adjustment algorithm may translate the additional partial feature data request into data acquisition instructions for the environment monitoring system. The data acquisition instructions may indicate to the environment monitoring system a desirable scan profile that may be capable of collecting the additional partial feature data. The data acquisition instructions may define suggested adjustments to a scan path including, but not limited to: Adjusting the rotational speed or other signal emission characteristics of a 360° scanning LIDAR system; defining a location or region on which to focus the scan direction; and/or adjusting/defining other scan characteristics.

The data acquisition instructions may be defined to account for the motion of the vehicle 300 in the design of the desirable scan path, whereby the settings and behavior of an adaptive scanning system may be adjusted relative to speed, direction and other motion data that is available to the vehicle 300 via its various sensors and control architectures.

For example, the data acquisition instructions may also provide suggestions to the wider vehicle 300 navigation system to facilitate acquisition of the additional partial feature data, including (but not limited to): adjusting the speed of the vehicle 300 to enable the environment monitoring system to acquire a more detailed scan; altering the motion of the vehicle 300, such as moving to a viable lane that is closer to objects in the gaze vector; and/or altering the vehicle's journey plan to facilitate a second or alternative drive-by of the gaze vector. In all of the above scenarios, the environment monitoring system and wider vehicle 300 navigation systems may prioritize the safety of the vehicle's passengers and other road users at all times. The data acquisition instructions may be incorporated into the vehicle's necessary navigational scanning behaviors only where it is safe to do so, as determined by the vehicle's controlling systems.

For example, the environment monitoring system may use the data acquisition instructions to adjust the scan path of one or more relevant sensors and acquire the additional feature data while the vehicle 300 continues its journey. The additional partial feature data may be collected from the feature of interest in real time, as the vehicle 300 passes it. LIDAR with a range of hundreds of meters may be capable of directing a scan from the rear of the vehicle 300, gathering sensor data for several seconds from an object that the vehicle 300 has already passed. The scan path adjustment algorithm may return the additional partial feature data to the feature extraction algorithm, where it is combined with the partial feature data to create the combined feature data or yet another partial feature data. The additional feature data and partial feature data may be combined by, for example, an algorithmic analysis and overlay of shared spatially distributed data points or larger features of the point cloud.

The method 100 may comprise adjusting 190 the motion of the vehicle 300 to facilitate monitoring 110 of the environment of the vehicle 300 based on the gaze direction of the passenger, thereby generating additional partial feature data for the feature of interest observed by the passenger. The method 100 may further comprise combining the partial feature data and the additional partial feature data, thereby updating the partial feature data for the feature of interest observed by the passenger.

As an example, adjusting 190 the motion of the vehicle 300 may comprise adjusting the speed of the vehicle 300. In so doing, monitoring 110 the environment of the vehicle 300 may be enabled to acquire a more detailed scan of the feature of interest. Adjusting the speed of the vehicle 300 may comprise decreasing the speed. Such may e.g. be beneficial when the feature of interest is stationary with respect to the moving vehicle 300. On the other hand, adjusting the speed of the vehicle 300 may comprise increasing the speed. Such may e.g. be beneficial when the feature of interest is moving away from the vehicle 300. Alternatively, or in addition, adjusting 190 the motion of the vehicle 300 may comprise altering the motion of the vehicle 300 so as to move to a location closer to the feature of interest (e.g. by choosing another viable lane of the road). Alternatively, or in addition, adjusting 190 the motion of the vehicle 300 may comprise altering the vehicle's journey plan to facilitate a second or alternative drive-by of the feature of interest (via a navigation system of the vehicle 300 and/or if the passengers agree). Any such adjusting 190 of the motion of the vehicle 300 may be instructed via a navigation system of the vehicle 300. In all of the above scenarios, adjusting 190 the motion of the vehicle 300 shall be subject to maintaining the safety of the vehicle 300 and other road users at all times. That is instructions for adjusting 190 the motion of the vehicle 300 shall only be accepted by the vehicle 300 where it is safe to do so, as determined by the vehicle's controlling system(s). Traffic rules shall be respected at all times.

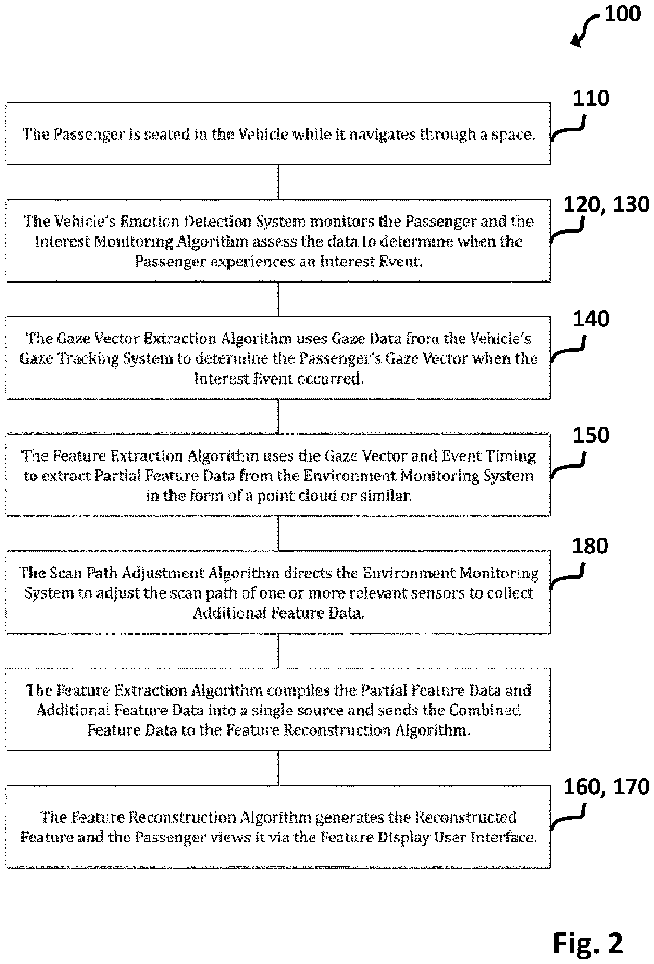

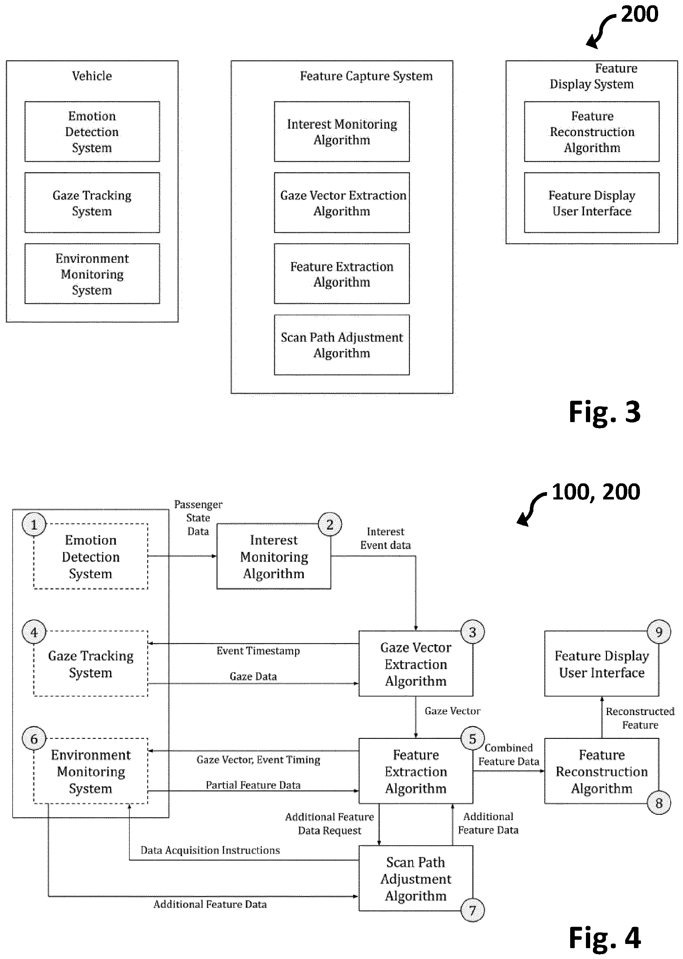

An exemplary embodiment of the computer-implemented method 100 for capturing a feature of interest observed by a passenger of a vehicle 300 is shown in **Fig. 2****.** An exemplary flow chart for a computer system 200 configured to run the method 100 is illustrated in **Fig. 4****.** Therein, the emotion detection system, the gaze tracking system, and the environment monitoring system may or may not be separate from the computer system 200. In case at least one of them is separate from the computer system 200, data therefrom may be obtained after sending an appropriate request according to a given interface/protocol.

There is disclosed a computer system 200 configured to execute the computer-implemented method 100 for capturing a feature of interest observed by a passenger of a vehicle 300. The computer system 200 may comprise at least one processor such as e.g. a (C)PU and a memory such as e.g. RAM. The computer system 200 may further comprise a storage such as e.g. HDD or SDD. The computer system 200 may be configured for data exchange with a (cloud) server. The computer system 200 may be an electronic control unit (ECU) in the vehicle 300. In examples, the computer system 200 may comprise a plurality of electronic control units (ECU) in the vehicle 300. The computer system 200 may comprise a/the user-interface. The computer system 200 may comprise (or be) the feature capture system of **Fig. 3****.** The computer system 200 may or may not comprise the feature display system. The computer system 200 may or may not comprise the emotion detection system, the gaze tracking system and/or the environment monitoring system. In case the latter systems are not comprised by the computer system 200, the computer system 200 may be configured to couple to the vehicle or these systems.

In case where steps 110 and/or 120 of the method 100 are carried out independently of the method 100, e.g. by an electronic control unit (ECU) of an autonomous driving control system of the vehicle 300, the computer system 200 may be configured to send requests for obtaining and/or to obtain environmental data generated by monitoring 110 the environment of the vehicle. Likewise, the computer system 200 may be configured to send requests for obtaining and/or to obtain passenger data generated by monitoring 120 the passenger of the vehicle. An exemplary architecture of the computer system 200 is illustrated in **Fig. 3** and **Fig. 4****.**

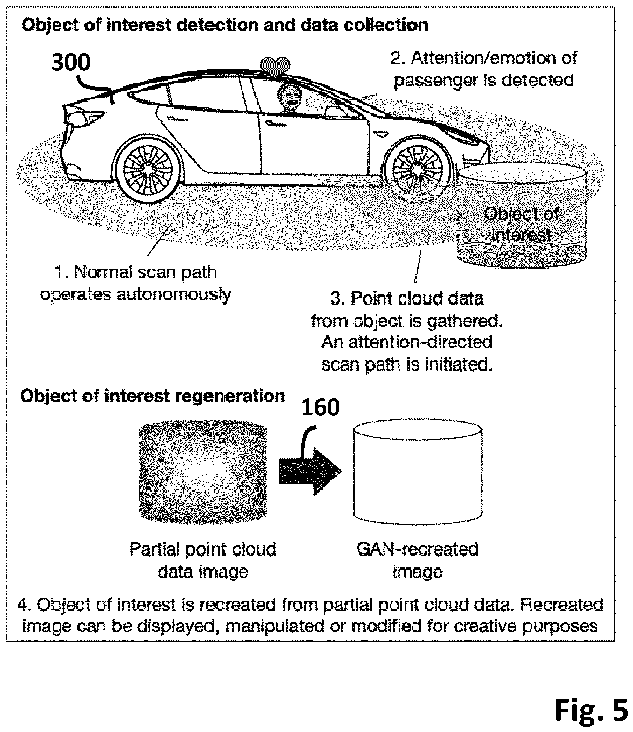

There is disclosed a vehicle 300, as for example illustrated in **Fig. 5****,** comprising an environment monitoring system configured to monitor 110 an environment of the vehicle 300, thereby generating environmental data. The vehicle 300 further comprises a passenger monitoring system configured to monitor 120 the passenger of the vehicle 300, thereby generating passenger data. The passenger monitoring system comprises an emotion detection system configured to detect 130 an excited level of the passenger's interest based on the passenger data, thereby generating an interest event. The passenger monitoring system further comprises a gaze tracking system configured to extract 140 a gaze direction of the passenger relating to the interest event based on the passenger data. The vehicle 300 may be configured to couple to the computer system 200. Alternatively, or in addition, the vehicle 300 may comprise the computer system 200.

There is disclosed a computer program configured to execute the computer-implemented method 100 for capturing a feature of interest observed by a passenger of a vehicle 300. The computer program may be in interpretable or compiled form. The computer program or portions thereof may be loaded as a bit or byte sequence into the RAM of the computer system 200.

There is disclosed a computer-readable medium or signal storing the computer program. The medium may be e.g. one of RAM, ROM, EPROM, HDD, SDD etc. storing the computer program.

There is disclosed a further computer-implemented method for recognizing a feature of potential interest to a passenger of a vehicle 300. The method comprises obtaining interest information for the passenger, wherein the interest information for the passenger is provided by the passenger and/or based at least on one feature of interest captured according to the computer-implemented method 100 for capturing the feature of interest observed by the passenger of the vehicle 300. The method further comprises monitoring an environment of the vehicle 300, thereby generating environmental data. The method further comprises testing, based on the interest information for the passenger, whether environmental data relate to a feature of potential interest to the passenger. The method further comprises recognizing the feature of potential interest, if testing is in the affirmative.

The method may comprise extracting the environmental data relating to the feature of potential interest, thereby generating partial feature data for the feature of potential interest. Steps 160, 170, 180, 190 of the computer-implemented method 100 for capturing the feature of interest observed by the passenger of the vehicle 300 may be analogously applied to the partial feature data for the feature of potential interest.

The method may comprise informing the passenger about the recognized feature of potential interest. Informing the passenger may comprise giving instructions to the passenger as to where the feature of potential interest is located.

The computer system 200 and/or the vehicle 300 may be configured run the computer-implemented method for recognizing a feature of potential interest to a passenger of a vehicle 300.

As an example, an algorithm (e.g. referred to as the predictive feature extraction algorithm) may pre-emptively gather partial feature data (e.g. referred to as the initial feature data) from the environment monitoring system, based on the passenger's historic interest event data.

The predictive feature extraction algorithm may be used to identify and pre-emptively collect data from features of interest, based on the passenger's previous interest events. Historic interest event data may be stored in a database (e.g. referred to as the interest event database) and assessed periodically by the predictive feature extraction algorithm to identify features that the user typically finds interesting (e.g. referred to as the predicted features). Alternatively, or in addition, the passenger may specify (e.g. via a user-interface) personal preferences.

Based on the historic interest event data and/or user-defined personal preferences, the predictive feature extraction algorithm may interface with the environment monitoring system to give early warning that a predicted feature is about to be observed.

When the environment monitoring system observes a predicted feature (likely before the passenger, via forward-facing long-range scanning), the scan path may be adjusted pre-emptively via the scan path adjustment algorithm to capture the maximum amount of feature data possible. As an example, existing machine vision technologies applied to output from the environment monitoring system may be used to monitor the data for predicted features. Although the present invention has been described above and is defined in the attached claims, it should be understood that the invention may alternatively be defined in accordance with the following embodiments:
1. A computer-implemented method (100) for capturing a feature of interest observed by a passenger of a vehicle (300), comprising:
   - detecting (130) an excited level of the passenger's interest based on passenger data generated by monitoring (120) the passenger of the vehicle (300), thereby generating an interest event;
   - extracting (140) a gaze direction of the passenger relating to the interest event based on the passenger data; and
   - extracting (150) environmental data, generated by monitoring (110) an environment of the vehicle (300), that relate to the interest event and to the gaze direction, thereby generating partial feature data for the feature of interest observed by the passenger.
2. The method (100) of embodiment 1, wherein:
   - monitoring (120) the passenger of the vehicle (300) comprises monitoring the passenger's emotional and/or psychological state, thereby generating passenger state data; and
   - detecting (130) the excited level of the passenger's interest is based on the passenger state data.
3. The method (100) of embodiment 1 or 2, wherein:
   - monitoring (120) the passenger of the vehicle (300) comprises monitoring the passenger's gaze direction, thereby generating passenger gaze data; and
   - extracting (140) the gaze direction of the passenger relating to the interest event based on the passenger gaze data.
4. The method (100) of one of the preceding embodiments, wherein detecting (130) the excited level of the passenger's interest based on the passenger data comprises:
   - applying the passenger data or a portion thereof relating to a current point in time to a predetermined criterion; and
   - detecting the excited level of the passenger's interest relating to the current point in time, if the predetermined criterion is satisfied;
   wherein the interest event comprises the current point of time.
5. The method (100) of embodiment 4, wherein the passenger data comprises data relating to one or more of a passenger's heart rate, a passenger's respiratory system, a passenger's pupillary response, a passenger's voice, and/or a passenger's speech, and wherein the excited level of the passenger's interest is detected (130), if one or more of the data relating to one or more of the passenger's heart rate, the passenger's respiratory system, the passenger's pupillary response, the passenger's voice, and/or the passenger's speech satisfy the predetermined criterion.
6. The method (100) of embodiment 4 or 5, wherein extracting (140) the gaze direction of the passenger relating to the interest event based on the passenger data comprises selecting a gaze direction from the generated passenger data relating to the current point in time.
7. The method (100) of one of the embodiments 4 to 6, wherein extracting (150) the environmental data that relate to the interest event and to the gaze direction comprises selecting a portion from the environmental data relating to the current point in time and the gaze direction.
8. The method (100) of one of the preceding embodiments, comprising:
   - reconstructing (160) the feature of interest observed by the passenger based on the partial feature data, thereby generating a digital representation of the feature of interest observed by the passenger.
9. The method (100) of embodiment 8, wherein reconstructing (160) the feature of interest observed by the passenger comprises applying the partial feature data to a pre-trained generative adversarial network (GAN) configured to output the digital representation of the feature of interest observed by the passenger.
10. The method (100) of embodiment 8 or 9, comprising:
   - visualizing (170) the digital representation of the feature of interest observed by the passenger via a user-interface.
11. The method (100) of one of the preceding embodiments, comprising:
   - adapting (180) the monitoring (110) of the environment of the vehicle (300) based on the gaze direction of the passenger, thereby generating additional partial feature data for the feature of interest observed by the passenger; and
   - combining the partial feature data and the additional partial feature data, thereby updating the partial feature data for the feature of interest observed by the passenger.
12. The method (100) of one of the preceding embodiments, comprising:
   - adjusting (190) the motion of the vehicle (300) to facilitate monitoring (110) of the environment of the vehicle (300) based on the gaze direction of the passenger, thereby generating additional partial feature data for the feature of interest observed by the passenger; and
   - combining the partial feature data and the additional partial feature data, thereby updating the partial feature data for the feature of interest observed by the passenger.
13. A computer system (200) configured to execute the computer-implemented method (100) for capturing a feature of interest observed by a passenger of a vehicle (300) according to one of the preceding embodiments.
14. A vehicle (300) comprising:
   - an environment monitoring system configured to monitor (110) an environment of the vehicle (300), thereby generating environmental data;
   - a passenger monitoring system configured to monitor (120) the passenger of the vehicle (300), thereby generating passenger data, wherein the passenger monitoring system comprises:
      - an emotion detection system configured to detect (130) an excited level of the passenger's interest based on the passenger data, thereby generating an interest event; and
      - a gaze tracking system configured to extract (140) a gaze direction of the passenger relating to the interest event based on the passenger data;
      wherein the vehicle (300) is configured to couple to or comprises the computer system (200) of embodiment 13.
15. A computer program configured to execute the computer-implemented method (100) for capturing a feature of interest observed by a passenger of a vehicle (300) according to one of the embodiments 1 to 12.
16. A computer-readable medium or signal storing the computer program of embodiment 15.
17. A computer-implemented method for recognizing a feature of potential interest to a passenger of a vehicle (300), comprising:
   - obtaining interest information for the passenger, wherein the interest information for the passenger is provided by the passenger and/or based at least on one feature of interest captured according to the computer-implemented method (100) for capturing the feature of interest observed by the passenger of the vehicle (300);
   - monitoring an environment of the vehicle (300), thereby generating environmental data;
   - testing, based on the interest information for the passenger, whether environmental data relate to a feature of potential interest to the passenger; and
   - recognizing the feature of potential interest, if testing is in the affirmative.
18. The method of embodiment 17, comprising:
   - extracting the environmental data relating to the feature of potential interest, thereby generating partial feature data for the feature of potential interest.
19. The method of embodiment 17 or 18, comprising:
   - informing the passenger about the recognized feature of potential interest.

## Claims

1. A computer-implemented method (100) for capturing a feature of interest observed by a passenger of a vehicle (300), comprising:
- detecting (130) an excited level of the passenger's interest based on passenger data generated by monitoring (120) the passenger of the vehicle (300), thereby generating an interest event;
- extracting (140) a gaze direction of the passenger relating to the interest event based on the passenger data; and
- extracting (150) environmental data, generated by monitoring (110) an environment of the vehicle (300), that relate to the interest event and to the gaze direction, thereby generating partial feature data for the feature of interest observed by the passenger.

2. The method (100) of claim 1, wherein:
- monitoring (120) the passenger of the vehicle (300) comprises monitoring the passenger's emotional and/or psychological state, thereby generating passenger state data; and
- detecting (130) the excited level of the passenger's interest is based on the passenger state data.

3. The method (100) of claim 1 or 2, wherein:
- monitoring (120) the passenger of the vehicle (300) comprises monitoring the passenger's gaze direction, thereby generating passenger gaze data; and
- extracting (140) the gaze direction of the passenger relating to the interest event based on the passenger gaze data.

4. The method (100) of one of the preceding claims, wherein detecting (130) the level of the passenger's interest based on the passenger data comprises:
- applying the passenger data or a portion thereof relating to a current point in time to a predetermined criterion; and
- detecting the level of the passenger's interest relating to the current point in time, if the predetermined criterion is satisfied;
wherein the interest event comprises the current point of time.

5. The method (100) of claim 4, wherein the passenger data comprises data relating to one or more of a passenger's heart rate, a passenger's respiratory system, a passenger's pupillary response, a passenger's voice, and/or a passenger's speech, and wherein the excited level of the passenger's interest is detected (130), if one or more of the data relating to one or more of the passenger's heart rate, the passenger's respiratory system, the passenger's pupillary response, the passenger's voice, and/or the passenger's speech satisfy the predetermined criterion.

6. The method (100) of claim 4 or 5, wherein extracting (140) the gaze direction of the passenger relating to the interest event based on the passenger data comprises selecting a gaze direction from the generated passenger data relating to the current point in time.

7. The method (100) of one of the claims 4 to 6, wherein extracting (150) the environmental data that relate to the interest event and to the gaze direction comprises selecting a portion from the environmental data relating to the current point in time and the gaze direction.

8. The method (100) of one of the preceding claims, comprising:
- reconstructing (160) the feature of interest observed by the passenger based on the partial feature data, thereby generating a digital representation of the feature of interest observed by the passenger.

9. The method (100) of claim 8, wherein reconstructing (160) the feature of interest observed by the passenger comprises applying the partial feature data to a pre-trained generative adversarial network (GAN) configured to output the digital representation of the feature of interest observed by the passenger.

10. The method (100) of claim 8 or 9, comprising:
- visualizing (170) the digital representation of the feature of interest observed by the passenger via a user-interface.

11. The method (100) of one of the preceding claims, comprising:
- adapting (180) the monitoring (110) of the environment of the vehicle (300) based on the gaze direction of the passenger, thereby generating additional partial feature data for the feature of interest observed by the passenger; and
- combining the partial feature data and the additional partial feature data, thereby updating the partial feature data for the feature of interest observed by the passenger.

12. The method (100) of one of the preceding claims, comprising:
- adjusting (190) the motion of the vehicle (300) to facilitate monitoring (110) of the environment of the vehicle (300) based on the gaze direction of the passenger, thereby generating additional partial feature data for the feature of interest observed by the passenger; and
- combining the partial feature data and the additional partial feature data, thereby updating the partial feature data for the feature of interest observed by the passenger.

13. A computer system (200) configured to execute the computer-implemented method (100) for capturing a feature of interest observed by a passenger of a vehicle (300) according to one of the preceding claims.

14. A vehicle (300) comprising:
- an environment monitoring system configured to monitor (110) an environment of the vehicle (300), thereby generating environmental data;
- a passenger monitoring system configured to monitor (120) the passenger of the vehicle (300), thereby generating passenger data, wherein the passenger monitoring system comprises:
- an emotion detection system configured to detect (130) an excited level of the passenger's interest based on the passenger data, thereby generating an interest event; and
- a gaze tracking system configured to extract (140) a gaze direction of the passenger relating to the interest event based on the passenger data;
wherein the vehicle (300) is configured to couple to or comprises the computer system (200) of claim 13.

15. A computer program configured to execute the computer-implemented method (100) for capturing a feature of interest observed by a passenger of a vehicle (300) according to one of the claims 1 to 12.
